# EUROPÄISCHE PATENTSCHRIFT

(11) **EP 2 657 216 B1**
(45) Veröffentlichungstag und Bekanntmachung des Hinweises auf die Patenterteilung: **25.06.2014**
(21) Anmeldenummer: 12165873.6
(22) Anmeldetag: 27.04.2012
(51) Int. Cl.: C07C 29/56, C07C 29/80, C07C 29/88, C07C 33/02

(54) **Verfahren zum Umsetzen von Farnesol zu Nerolidol in Gegenwart von alpha-Bisabolol**
Method for converting farnesol to nerolidol in the presence of alpha-bisabolol
Procédé de basculement du farnésol au nérolidol en présence d'alpha-bisabolol

(43) Veröffentlichungstag der Anmeldung: 30.10.2013
(73) Patentinhaber: Symrise AG, 37603 Holzminden (DE)
(72) Erfinder: Siewert, Jürgen, 37434 Rollshausen (DE); Wilkening, Burghard, 37619 Bodenwerder (DE)
(74) Vertreter: Eisenführ Speiser

(56) Entgegenhaltungen:
- WO-A1-2007/082847
- DE-A1-102005 051 903
- DE-A1-102005 053 338

## Beschreibung

Die vorliegende Erfindung betrifft ein Verfahren zum Umsetzen von Farnesol zu Nerolidol in Gegenwart von alpha-Bisabolol. Somit betrifft die vorliegende Erfindung ein Verfahren zur Abreicherung von Farnesol aus Mischungen enthaltend Farnesol und alpha-Bisabolol.

Natürliches alpha-Bisabolol ist ein wichtiger Bestandteil des ätherischen Öls der Kamillenart Chamomilla recutita. In kosmetischen und pharmazeutischen Anwendungen wird alpha-Bisabolol aufgrund seiner hautberuhigenden und entzündungshemmenden Eigenschaften eingesetzt. Weiterhin wird alpha-Bisabolol in der Riechstoffindustrie als Geruchsstoff eingesetzt.

Während der systematische Anbau von Arznei- und Gewürzpflanzen wie Kamille aufgrund einer gestiegenen Nachfrage nach "nachwachsenden Rohstoffen" weiterhin an Bedeutung gewinnt, führten die beschränkten natürlichen Ressourcen gleichzeitig zu der Suche und Entwicklung von Verfahren zur Gewinnung synthetischer Produkte.

Synthetisches "alpha-Bisabolol" stellt üblicherweise ein diastereomeres Racemat aus gleichen Anteilen (+/-)-alpha-Bisabolol und (+/-)-epi-alpha-Bisabolol dar. Diese vier Enantiomeren (+)-epi-alpha-Bisabolol, (-)-alpha-Bisabolol, (+)-epi-alpha-Bisabolol, (-)-epi-alpha-Bisabolol besitzen eine Struktur gemäß der Formel A 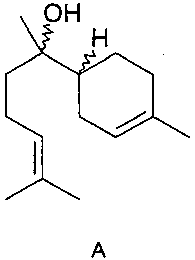 in der geschlängelte Linien jeweils unabhängig voneinander für eine S- oder R-Konfiguration am zugehörigen C-Atom stehen. Die Verbindungen der Formel **A** werden im vorliegenden Text zusammenfassend mit dem Begriff alpha-Bisabolol bezeichnet.

Aufgrund ihrer strukturellen Ähnlichkeit zu alpha-Bisabolol bieten sich die Sesquiterpene Nerolidol (Formel **B**) und Farnesol (Formel **C**), in der geschlängelte Linien jeweils unabhängig voneinander für eine S- oder R-Konfiguration am zugehörigen C-Atom stehen, als Ausgangsmaterialien für die industrielle Synthese an.

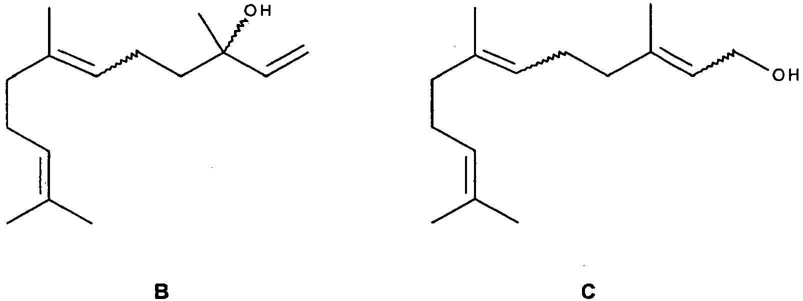

So wurden in der Vergangenheit eine Vielzahl von Verfahren und Prozessen zur Herstellung von alpha-Bisabolol ausgehend von Nerolidol oder Farnesol beschrieben.

Die erste katalytische Cyclisierung von Farnesol wurde 1913 beschrieben, als man beobachtete, dass bei einer Reaktionsausführung in Anwesenheit von Kaliumhydrogensulfat neben den erwarteten Kohlenwasserstoffen auch einige mono- und bicyclische Verbindungen gefunden wurden [Chem. Ber. 46, 4024 (1913)]. Spätere Arbeiten identifizierten dann diese cyclischen Verbindungen als Verbindungen der Bisabolen- und Cadalen-Klasse.

1925 wurde erstmals gezeigt, dass ausgehend von Nerolidol durch Säurekatalyse Produkte wie Farnesen, Bisabolen und alpha-Bisabolol erhalten werden [Helv. Chim. Acta 8, 259 (1925)]. Es wurde insbesondere gezeigt, dass Nerolidol durch Zusatz von Acetanhydrid, anschließenden Umsatz mit Essigsäure/Schwefelsäure oder Ameisensäure bei Raumtemperatur und anschließende Verseifung ein Gemisch liefert, welches alpha-Bisabolol und Farnesol umfasst.

1968 berichtet Gutsche [Tetrahedron 24, 859] über die säurekatalysierte Cyclisierung von Farnesol und Nerolidol. Ausgehend von Farnesol oder Nerolidol wurden zunächst durch Umsetzen mit Ameisensäure die entsprechenden Formiate erhalten, die dann in einem zweiten Schritt zu den Alkoholen verseift wurden. Nach dieser Vorgehensweise entstehen jedoch Substanzgemische, die neben alpha-Bisabolol auch Farnesol enthalten. Eine anschließende destillative Aufreinigung in hochangereichertes alpha-Bisabolol erweist sich als schwierig, insbesondere weil alpha-Bisabolol und cis, cis-Farnesol nahezu identische Siedepunkte aufweisen und die nach der beschriebenen Vorgehensweise erhaltenen Substanzgemische bis zu 10% cis, cis-Farnesol enthalten.

Weitere Synthesen von alpha-Bisabolol wurden von Ruzicka et. al. [Helv. Chim. Acta 15, 3, (1932)] und durch Manjarrez et. al. [J. Org. Chem. 31, 348, (1966)] beschrieben. Die säurekatalysierte Cyclisierung in Gegenwart von Ameisensäure in Pentan bzw. AlCl₃ in Ether [Tetrahedron Lett. 1972, 2455], KHSO4 [J. Org. Chem. 34, 3789, (1969)] und BF3-Etherat in Methylenchlorid [Chem. Lett. 1972, 263] wurde ebenfalls beschrieben.

Uneyama et. al. berichten über eine elektrochemische Darstellungsmethode [Chem. Lett. 1984, 529], dabei wird auch über die Herstellung von DL-Bisabolol aus DL-Nerolidol berichtet. Während die zuvor vorgestellten von Nerolidol ausgehenden Verfahren selten zu alpha-Bisabolol-Ausbeuten über 30% führten, wurden mit elektrochemischen Verfahren Ausbeuten bis zu 52% erhalten.

WO 2004/033401 beschreibt ein Verfahren zur Herstellung von alpha-Bisabolol, worin Nerolidol in einer Stufe mit einem Gemisch bestehend aus einem Keton, einer Sulfonsäure und Perchlorsäure umgesetzt wird. Dieses Verfahren zeichnet sich unter anderem dadurch aus, dass es zu einem besonders reinen alpha-Bisabolol führt und insbesondere das bei den zuvor beschriebenen Verfahren als Nebenprodukt in einer Ausbeute von bis zu 40 % entstehende (+), (-) oder (+/-)-Farnesol nur in vergleichsweise geringen Konzentrationen entsteht. Der Einsatz von Perchlorsäure ist aus sicherheitstechnischen Gründen jedoch problematisch.

Der aus sicherheitstechnischer Sicht kritische Einsatz von Perchlorsäure wird in dem aus DE 10 2005 053 338 bekannten Verfahren zur Herstellung von alpha-Bisabolol, umfassend die Umsetzung von Farnesol oder Nerolidol oder Gemischen von Farnesol und Nerolidol in Gegenwart eines Ketons, einer Sulfonsäure und einer weiteren starken Säure, ausgenommenen Perchlorsäure, vermieden.

US 2008/0269530 A1 beschreibt ein Verfahren zur Herstellung von alpha-Bisabolol, umfassend die Umsetzung von Farnesol in Gegenwart eines Ketons, einer Sulfonsäure und einer weiteren starken Säure. Das resultierende Produktgemisch enthält alpha-Bisabolol und dessen Dehydratisierungprodukte als Hauptkomponenten. Farnesol ist nach Aufarbeitung des Reaktionsgemischs nur noch in geringen Konzentrationen enthalten.

Synthesen ausgehend von Farnesol liefern das gewünschte alpha-Bisabolol jedoch nur in geringeren Ausbeuten im Vergleich zu den von Nerolidol ausgehenden Synthesen.

Den bekannten Verfahren zur Herstellung von alpha-Bisabolol ist gemeinsam, dass dort regelmäßig in mehr oder weniger großen Mengen auch Farnesol entsteht. Die Anwesenheit von Farnesol in Produktgemischen neben alpha-Bisabolol ist jedoch unerwünscht, weil Farnesol ein allergenes Potential zugeschrieben wird, welches insbesondere die Verwendung in kosmetischen Produkten problematisch macht. Bei der Entwicklung von Kosmetikprodukten sind nämlich nicht allein die kosmetischen Eigenschaften von Interesse, sondern es muss selbstverständlich auch die Unbedenklichkeit der enthaltenen Substanzen gegenüber Mensch und Umwelt gewährleistet sein. Zum Wert eines neuen Produktes tragen insbesondere verbesserte toxikologische, ökotoxikologische und dermatologische Eigenschaften bei. Aus dermatologischer Sicht soll ein Kosmetikprodukt keine hautreizenden, sensibilisierenden und/oder photosensibilisierenden Eigenschaften aufweisen. Insoweit wird die Anwesenheit von Farnesol in Kosmetikprodukten in zunehmendem Maße als problematisch empfunden.

Es wurde bereits oben erwähnt, dass die destillative Trennung von alpha-Bisabolol und Farnesol insbesondere deshalb schwierig ist, weil alpha-Bisabolol und cis,cis-Farnesol über nahezu identische Siedepunkte verfügen. Wenn Produktgemische, die neben dem erwünschten alpha-Bisabolol auch einen nennenswerten Anteil an Farnesol umfassen, dennoch destillativ aufgetrennt werden sollen, so ist zum Erreichen zumindest eines gewissen Erfolges eine derart lange thermische Belastung erforderlich, dass es zu einem hohen Maße an Nebenreaktionen und insbesondere zur Zersetzung der zuvor synthetisierten Verbindungen kommt. Zusätzlich liefert die herkömmliche destillative Trennung von alpha-Bisabolol und Farnesol nur Farnesol von minderer Reinheit, welches nicht zur direkten Weiterverwendung geeignet ist, insbesondere nicht als Edukt für die Synthese von alpha-Bisabolol.

Die Entfernung von Farnesol aus Gemischen mit alpha-Bisabolol durch Veresterung wird in US 7,399,880 B2 beschrieben. Das Verfahren umgeht das destillative Trennproblem durch die selektive Umesterung des in der Mischung enthaltenen Farnesols mit einem Carbonsäureester in Gegenwart eines Umesterungskatalysators. Die so erhaltenen Farnesylester weisen nun Siedepunkte auf, welche sich deutlich von denen der alpha-Bisabolole unterscheiden, und können durch einfache Destillation abgetrennt werden. Nach diesem Verfahren können alpha-Bisabolol enthaltende Produktgemische mit Gehalten an Farnesol kleiner 0,5% erreicht werden.

Die selektive Veresterung von Farnesol wird gemäß DE 10 2005 026 768 durch Umsetzung des in der Mischung enthaltenen Farnesol mit einer Stickstoffbase und einem Benzoylhalogenid erreicht. Hierbei entstehen selektiv die entsprechenden Farnesylbenzoate, welche in einem einfachen Destillationsschritt abgetrennt werden können.

In US 2010/0222606 A1 (WO 2007/082847) wird das Problem der Trennung von Farnesol und alpha-Bisabolol ebenfalls durch Veresterung des Farnesols gelöst. Das Verfahren zeichnet sich insbesondere dadurch aus, dass keine Abwässer anfallen. Die als Koppelprodukt anfallenden Farnesylcarbonsäureester können z.B. für Riechstoffanwendungen verwendet, oder, in einem alternativen Schritt, durch Verseifung in Farnesol überführt werden. Hierbei fällt jedoch Prozessabwasser an. Das so erhaltene Farnesol kann als Edukt zur Herstellung von alpha-Bisabolol verwendet werden

Es war die Aufgabe der vorliegenden Erfindung, ein Verfahren anzugeben, welches ein selektives Umsetzen von Farnesol in Gegenwart von alpha-Bisabolol erlaubt, so dass ein alpha-Bisabolol enthaltendes Produktgemisch erhalten werden kann, das weitgehend oder im Wesentlichen frei von Farnesol ist. Vorzugsweise sollte das Farnesol zu einem Produkt umgesetzt werden, welches seinerseits in einfacher Weise mit guten Ausbeuten zu alpha-Bisabolol umgesetzt werden kann.

Diese Aufgabe wird erfindungsgemäß gelöst durch ein Verfahren zum Umsetzen von Farnesol zu Nerolidol in Gegenwart von alpha-Bisabolol, mit folgenden Schritten:
- Bereitstellen oder Herstellen einer Mischung umfassend
   - alpha-Bisabolol,
   - Farnesol,
   - einen oder mehrere Katalysatoren zur selektiven Isomerisierung von Farnesol zu Nerolidol in Gegenwart von alpha-Bisabolol
   - sowie gegebenenfalls weitere Bestandteile,
- Umsetzen von Farnesol zu Nerolidol in der Mischung.

Der Begriff "alpha-Bisabolol" umfasst dabei im Rahmen dieses Textes (+)-alpha-Bisabolol, (-)-alpha-Bisabolol, (+)-epi-alpha-Bisabolol und (-)-epi-alpha-Bisabolol sowie Mischungen von zwei, drei oder sämtlichen der genannten Isomeren des alpha-Bisabolols. Insbesondere umfasst der Begriff "alpha-Bisabolol" racemische Gemische von (+/-)-alpha-Bisabolol und/oder (+/-)-epi-alpha-Bisabolol.

Der Begriff "Farnesol" umfasst dabei im Rahmen dieses Textes das cis/cis-, das cis/trans-, das trans/cis- und das trans/trans-Isomer sowie Mischungen von zwei, drei oder sämtlichen der genannten Isomeren des Farnesols.

Die Erfindung beruht auf der überraschenden Erkenntnis, dass das in der her- oder bereitgestellten Mischung umfassend alpha-Bisabolol, Farnesol und gegebenenfalls weitere Bestandteile enthaltene Farnesol sich in Gegenwart eines geeigneten Katalysators selektiv zu Nerolidol (Siedepunkt 145 bis 146 °C bei 16 mbar) umsetzen lässt, welches mit geringem destillativem Aufwand vom alpha-Bisabolol abgetrennt werden kann. Am Reaktionsende liegt ein Produktgemisch umfassend alpha-Bisabolol, Nerolidol und einen gegenüber der her- oder bereitgestellten Mischung verminderten Anteil an Farnesol vor.

Erfindungsgemäß werden die Verfahrensbedingungen vorzugsweise so gewählt, dass nach dem Umsetzen von Farnesol zu Nerolidol das Molverhältnis von alpha-Bisabolol zu Farnesol größer ist als in der bereitgestellten oder hergestellten Mischung.

In der bereitgestellten oder hergestellten Mischung liegt das Molverhältnis von alpha-Bisabolol zu Farnesol bevorzugt in einem Bereich von 15:1 bis 1:100, vorzugsweise 2 : 1 bis 1 : 2. Das Molverhältnis und die jeweilige Stoffmenge in der Mischung lässt sich beispielsweise mittels Gaschromatographie bestimmen, wobei zur präzisen Bestimmung ein interner Standard einzusetzen ist. Sofern nachfolgend nicht anders angegeben, ist diese Bestimmungsmethode anzuwenden.

Bei Abwesenheit eines internen Standards kann das GC-Flächenverhältnis verglichen und mit dem Molverhältnis gleichgesetzt werden; hierbei entsteht üblicherweise ein geringfügiger Fehler, der jedoch im Allgemeinen in der industriellen Praxis zu vernachlässigen oder akzeptabel ist.

Erfindungsgemäß bevorzugt werden die Verfahrensbedingungen so gewählt, dass nach dem Umsetzen von Farnesol zu Nerolidol das Molverhältnis von alpha-Bisabolol zu Farnesol 30 : 1 oder höher ist, bevorzugt 50 : 1 oder höher, weiter bevorzugt 80 : 1 oder höher, besonders bevorzugt 100 : 1 oder höher. Bevorzugte Ausgestaltungen des erfindungsgemäßen Verfahrens werden vorzugsweise miteinander kombiniert.

Die erfindungsgemäße katalytische Umsetzung von Farnesol (C) zu Nerolidol (B) in Gegenwart von alpha-Bisabolol (A), das selbst nicht oder nur in geringem Maße umgesetzt wird, ist ebenso wie das erfindungsgemäß bevorzugte Abdestillieren des Nerolidol im folgenden Schema 1 vereinfacht dargestellt. Dabei sind die gezeigten Isomere des alpha-Bisabolols, des Farnesols und des Nerolidols lediglich beispielhaft zu verstehen. 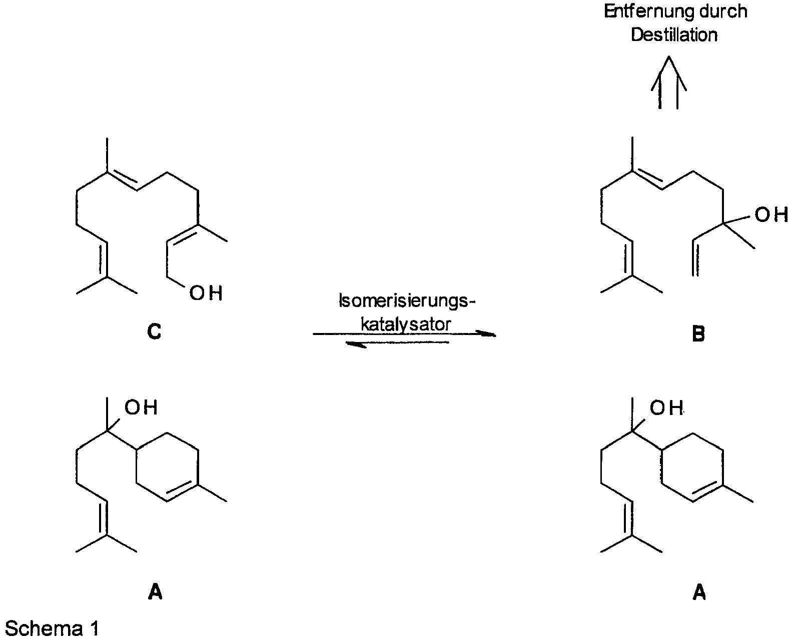 Die Umlagerung von höheren Allylalkoholen ist aufgrund der vielfältigen Verwendung dieser Allylalkohole, z.B. bei der Produktion von Weichmacheralkoholen oder der Vitamine A und E, ein wirtschaftlich relevantes Gebiet, insbesondere da die säurekatalysierte Umlagerung oft nur unter schlechten Ausbeuten erfolgt. Im Stand der Technik finden sich daher verschiedene Lösungsansätze, welche vorrangig die Isomerisierung von tertiären Allylalkoholen zu den primären oder sekundären Allylalkoholen behandeln, insbesondere das Isomerenpaar Linalool und Nerol / Geraniol.

In GB 1 256 184 (US 3,925,485) wird die Isomerisierung von Allylalkoholen unter Verwendung von katalytischen Mengen von Übergangsmetallverbindungen der Gruppen 5, 6 und 7 beschrieben. Dabei werden Vanadiumverbindungen bevorzugt. Beispiele für die Verwendung von Verbindungen anderer Elemente werden nicht angegeben.

US 4,006,193 beschreibt die Verwendung von Verbindungen von Übergangsmetallen der Gruppen 5, 6 und 7. Auch hier werden die Vanadiumverbindungen bevorzugt, da diese im Vergleich zu den Molybdän, Rhenium und Chromverbindungen deutlich bessere Ausbeuten und Selektivitäten ermöglichen. Die destillative Isomerisierung von Farnesol zu Nerolidol wird hierbei als Beispiel aufgeführt.

In DE 25 16 698 wird die Verwendung von verbesserten Wolfram-Komplexen beschrieben. Die Komplexe weisen Silanol-Gruppen sowie eine Stickstoff-haltigen Base als Liganden auf.

In der akademischen Literatur wurden die vorhergehend genannten Katalysatorsysteme in Chem. Lett. 1982, 357-360 sowie Tetrahedron 1977, 33, 1775-1783 beschrieben.

Neben den bereits beschriebenen katalysierten Allylumlagerungen mit Komplexen und Verbindungen von Übergangsmetallen der Gruppen 5, 6 und 7 beschreibt DE 25 57 837 zusätzlich die Anwendbarkeit von Verbindungen von Übergangsmetallen der Gruppe 8 sowie die Möglichkeit der Allylumlagerung unter Verwendung eines Katalysatorsystems bestehend aus einer organischen oder anorganischen Schwefelverbindung und eines Radikalstarters.

EP 0 585 680 (US 5,349,097) betrifft die Allylumlagerung unter Verwendung von Rhenium-Verbindungen. Unter Verwendung von Organo-Rhenium-Katalysatoren, wie z.B. Methyltrioxorhenium (MTO), konnten deutlich bessere Ergebnisse im Vergleich zu den bisher beschriebenen Rhenium-Verbindungen erreicht werden.

DE 100 46 865 beschreibt die Verwendung eines Peroxowolfram-Komplexes als Katalysator für die Isomerisierung von Allylalkoholen. Vergleichbar zu DE 25 16 698 wird auch hier eine Stickstoff-Base, speziell 8-Hydroxychinolin, als Ligand verwendet.

WO 2008/098774 beschreibt eine Kombination aus einem Übergangsmetall, dem Oxidans TEMPO (2,2',6,6'-Tetramethylpiperidin-1-oxyl) und einem Co-Oxidans als effektiven Katalysator für die Allylumlagerung.

Weitere Katalysatoren für die Isomerisierung von Allylalkoholen sind aus US 6 566 564 und US 6 989 468 bekannt.

Bevorzugt umfasst in erfindungsgemäßen Verfahren einer, mehrere oder sämtliche der Katalysatoren zur selektiven Isomerisierung von Farnesol zu Nerolidol in Gegenwart von alpha-Bisabolol eine Verbindung eines Übergangsmetalls der Gruppen 5 bis 8 des Periodensystems, vorzugsweise eine Verbindung eines Übergangsmetalls ausgewählt aus der Gruppe bestehend aus Vanadium, Rhenium, Wolfram, Mangan, Chrom, Molybdän und Palladium. In eigenen Untersuchungen wurde festgestellt, dass solche Katalysatoren bei Zusatz zu einem Gemisch umfassend alpha-Bisabolol und Farnesol selektiv das Farnesol zu Nerolidol umsetzen, während das alpha-Bisabolol unter den Reaktionsbedingungen stabil ist, bzw. nur geringfügig umgesetzt bzw. zersetzt wird.

Besonders bevorzugt sind bei dem erfindungsgemäßen Verfahren einer, mehrere oder sämtliche der Katalysatoren zur selektiven Isomerisierung von Farnesol zu Nerolidol in Gegenwart von alpha-Bisabolol ausgewählt aus der Gruppe bestehend aus:
- Vanadiumsäure und deren Ester und Ammoniumsalze,
- Chelate und Organometallverbindungen der Übergangsmetalle der Gruppen 5 bis 8 des Periodensystems, vorzugsweise Chelate und Organometallverbindungen der Übergangsmetalle ausgewählt aus der Gruppe bestehend aus Vanadium, Rhenium, Wolfram, Mangan, Chrom, Molybdän und Palladium.

Besonders bevorzugt sind Ester (VO(OR¹)(OR²)(OR³)) der Orthovanadiumsäure und Chelate (VO(R⁴(C-O)CH(C=O)R⁵)₂) des vierwertigen Vanadiums und Chelate (V(R⁴(C-O)CH(C=O)R⁵)₃) des dreiwertigen Vanadiums, wobei hierin R¹, R², R³, R⁴, R⁵ jeweils einen organischen Rest bedeuten und wobei R¹, R², R³, R⁴, R⁵ unabhängig voneinander vorzugsweise jeweils einen oder mehrere Arylreste mit maximal 20 C-Atomen oder verzweigte- oder geradkettige Alkylreste enthalten, die unabhängig voneinander jeweils 1 bis 20 C-Atome besitzen. Die Reste R¹, R², R³, R⁴, R⁵ können ebenfalls einfach oder mehrfach ungesättigt sein.

Zudem besonders bevorzugt sind Wolfram-(VI)-oxoalkoholate (WO(OR¹)(OR²)(OR³) (OR⁴)), wobei die Reste R¹, R², R³, R⁴ jeweils einen organischen Rest bedeuten und vorzugsweise jeweils unabhängig voneinander einen Arylrest mit maximal 20 C-Atomen oder verzweigten oder geradkettigen Alkyl- oder (einfach oder mehrfach ungesättigten) Alkenylrest mit 1 bis 20 C-Atomen bedeuten.

Ebenfalls besonders bevorzugt sind Wolfram-(VI)-oxoperoxo-Komplexe [(WO(O₂)L¹L²) oder (WO(O₂)₂L¹L²)], wobei L¹ und L² unabhängig voneinander Alkoxy-Liganden (OR⁶) oder Amino-Liganden (N(R⁷)(R⁸)(R⁹)) bedeuten. Die Reste R⁶, R⁷, R⁸, R⁹ sind hierbei organische Reste, vorzugsweise unabhängig voneinander jeweils Arylreste mit maximal 20 C-Atomen oder geradkettige oder verzweigte Alkyl- oder (einfach oder mehrfach ungesättigte) Alkenylreste mit 1-20 C-Atomen. Die Liganden L¹ und L² können alternativ in Form eines Chelat-Liganden, d.h. untereinander verknüpft, vorliegen, wie z.B. in 8-Hydroxychinolin.

Ganz besonders bevorzugt für das erfindungsgemäße Verfahren sind Katalysatoren ausgewählt aus der Gruppe bestehend aus Vanadium(III)-acetylacetonat, Vanadyl(IV)-acetylacetonat, Vanadium(V)-oxytriisopropoxid, Ammoniummetavanadat und Oxoperoxowolfram(VI)-Komplexen umfassend einen oder mehrere Liganden in Form von 8-Hydroxychinolin.

Die Herstellung von Oxoperoxowolfram(VI)-Komplexen umfassend einen oder mehrere Liganden in Form von 8-Hydroxychinolin wird in DE 100 46 865 A1 beschrieben, man vergleiche dort insbesondere die Absätze [0057] bis [0062].

Bei dem erfindungsgemäßen Verfahren wird der Fachmann für das jeweilige Katalysatorsystem die Reaktionsbedingungen so modifizieren, dass die Stabilität des in der Reaktionsmischung vorliegenden alpha-Bisabolols gewährleistet ist. Insbesondere sind hier die Reaktionstemperaturen, sowie Liganden und Lösungsmittel zur Modifizierung der Lewis-Acidität der jeweiligen Übergangsmetallverbindung als vom Fachmann einzustellende Parameter zu nennen.

Bei dem erfindungsgemäßen Verfahren ist es bevorzugt, dass die bereitgestellte oder hergestellte Mischung 0,0001 bis 1 Mol, vorzugsweise 0,001 bis 0,5 Mol an Übergangsmetall pro Mol Farnesol, enthält, bezogen auf die in der bereitgestellten oder hergestellten Mischung vorhandene Stoffmenge an Farnesol.

Erfindungsgemäß bevorzugt erfolgt das Umsetzen von Farnesol zu Nerolidol bei einer Temperatur im Bereich von 50 bis 300 °C, vorzugsweise im Bereich von 150 bis 200 °C. und/oder das Umsetzen wird bei einem Druck von weniger als 1000 mbar, vorzugsweise im Bereich vom 0,1 bis 500 mbar durchgeführt.

Typischerweise wird das bei der Umsetzung von Farnesol entstehende Nerolidol während und/oder nach der Umsetzung durch Rektifikation aus dem Reaktionsgemisch entfernt.

Die Reaktionsführung kann erfindungsgemäß sowohl absatzweise (batch-Fahrweise), als auch kontinuierlich oder teilkontinuierlich erfolgen.

In einer besonders bevorzugten erfindungsgemäßen Verfahrensausgestaltung wird eine Mischung umfassend alpha-Bisabolol, Farnesol sowie gegebenenfalls weitere Bestandteile mit einem oder mehreren Katalysatoren zur selektiven Isomerisierung von Farnesol zu Nerolidol in Gegenwart von alpha-Bisabolol versetzt. Die bereitgestellte Mischung wird dann erhitzt und das entstehende Nerolidol destillativ unter Verwendung einer Rektifikationskolonne abgetrennt. Auf diese Weise ließen sich hervorragende Ergebnisse erzielen. Zu in diesem Fall bevorzugten Katalysatoren sei auf die obigen Angaben vorwiesen.

In der erfindungsgemäß bereitgestellten oder hergestellten Mischung sind gegebenenfalls ein oder mehrere weitere Bestandteile vorhanden, die beispielsweise ausgewählt sind aus der Gruppe bestehend aus: Nerolidol, Eliminierungsprodukte des alpha-Bisabolols, Eliminierungsprodukte des Farnesols, Eliminierungsprodukte des Nerolidols, Veretherungsprodukte des Farnesols, weitere Sesquiterpene, weitere Sesquiterpenalkohole, weitere Umlagerungsprodukte von alpha-Bisabolol, Nerolidol und Farnesol, sonstige Lösungsmittel. Bei den Eliminierungsprodukten des alpha-Bisabolols handelt es sich insbesondere um Bisabolene. Bei den Eliminierungsprodukten des Farnesols handelt es sich insbesondere um Farnesene. Bei den Veretherungsprodukten des Farnesols handelt es sich insbesondere um Difarnesylether. Bei den Sesquiterpenalkoholen handelt es sich insbesondere um Sesquiterpenalkohole aus der Gruppe bestehend aus Khusiol, Germacradienol, Elemol, Eudesmol und beta-Bisabolol.

Dabei ist die Zusammensetzung der her- oder bereitgestellten Mischung üblicherweise abhängig von dem Verfahren, mittels dessen das in der Mischung enthaltene alpha-Bisabolol und Farnesol gebildet wurde.

In einer bevorzugten Variante enthält die her- oder bereitgestellte Mischung ein oder mehrere Lösungsmittel, die unter den Reaktionsbedingungen stabil sind und die katalytische Aktivität des Katalysators nicht negativ beeinflussen. Geeignete Lösungsmittel sind hochsiedende Lösungsmittel. Vorteilhafterweise eingesetzte hochsiedende Lösungsmittel haben gemein, daß ihr Siedepunkt über dem des alpha-Bisabolols liegt, was eine spätere destillative Abtrennung des alpha-Bisabolols vom Lösungsmittel ermöglicht. Bevorzugte hochsiedende Lösungsmittel sind ausgewählt aus der Gruppe bestehend aus:
- Ether, wie z.B. Polyethylenglycolether oder Polypropylenglycolether
- ein- und zweiwertige Alkohole wie z.B. Palmitylalkohol, Stearylalkohol sowie Diethylenglykol und Triethylenglykol
- Polyethylenglycole und Polypropylenglycole.
- Sulfoxide wie z.B. Sulfolan
- Kohlenwasserstoffe wie z.B. Squalan oder Paraffinöle eingesetzt werden.

Die Menge des verwendeten Lösungsmittels liegt vorzugsweise im Bereich von 5 bis 100 Gew.-% bezogen auf die Gesamtmasse der her- oder bereitgestellten Mischung.

In dem erfindungsgemäßen Verfahren umfasst der Schritt des Herstellens der Mischung vorzugsweise das Umsetzen von Nerolidol zu einer alpha-Bisabolol und Farnesol umfassenden Mischung Dabei wird bevorzugt eine Mischung umfassend alpha-Bisabolol, Farnesol sowie gegebenenfalls weitere Bestandteile nach einem der oben erwähnten Verfahren hergestellt, vorzugsweise nach dem Verfahren gemäß WO 2004/033401. Alternative Verfahren zur Herstellung von alpha-Bisabolol, insbesondere solche, die von Nerolidol als Edukt ausgehen, können ebenfalls benutzt werden.

Im Rahmen des erfindungsgemäßen Verfahrens umfasst der Schritt des Herstellens der Mischung bevorzugt das
- Verseifen von alpha-Bisabolylformiat und Farnesylformiat in Mischung miteinander oder
- Umestern von alpha-Bisabolylformiat und Farnesylformiat in Mischung miteinander mit einem Alkohol.

Dabei entsteht jeweils eine Produktmischung umfassend alpha-Bisabolol und Farnesol.

In einer bevorzugten Variante des erfindungsgemäßen Verfahrens werden während und/oder nach dem Umsetzen des Farnesols zu Nerolidol alpha-Bisabolol und Nerolidol voneinander getrennt. Bevorzugt erfolgt während und/oder nach dem Umsetzen des Farnesols zu Nerolidol ein destillatives Abtrennen des Nerolidols aus der durch Umsetzen des Farnesols zu Nerolidol erhaltenen Mischung.

Vorzugsweise wird danach das gewünschte Produkt alpha-Bisabolol bei einem Druck im Bereich von 0,1 - 5 mbar über Kopf destilliert. Diese Destillation erfolgt ebenfalls absatzweise (batch-Fahrweise) oder kontinuierlich, z.B. unter Verwendung eines Dünnschichtverdampfers. Im Sumpf verbleiben der Katalysator und gegebenenfalls weitere Bestandteile mit einem Siedepunkt, der höher liegt als der Siedepunkt von alpha-Bisabolol.

In einer besonders bevorzugten Variante des erfindungsgemäßen Verfahrens werden die Schritte
- Herstellen einer Mischung umfassend
   - alpha-Bisabolol,
   - Farnesol,
   - einen oder mehrere Katalysatoren zur selektiven Isomerisierung von Farnesol zu Nerolidol in Gegenwart von alpha-Bisabolol
   - sowie gegebenenfalls weitere Bestandteile
und
- Umsetzen von Farnesol zu Nerolidol in der Mischung

in demselben Reaktionsgefäß ausgeführt. Das Herstellen einer Mischung umfassend alpha-Bisabolol und Farnesol sowie das Umsetzen von Farnesol zu Nerolidol in der Mischung erfolgt in situ als Eintopf-Reaktion ohne Isolieren der hergestellten Mischung umfassend alpha-Bisabolol und Farnesol. Bei dieser Variante des erfindungsgemäßen Verfahrens ist es besonders vorteilhaft, wenn der Schritt des Herstellens der Mischung im besagten Reaktionsgefäß das Umestern von Bisabolylformiat und Farnesylformiat mit einem Alkohol R¹⁰-OH erfasst. Der Rest R¹⁰ kann hierbei ein C1-C6-Alkylrest, wie z.B. Methyl, Ethyl, Propyl, Cylcopropyl, 1-Methylethyl, Butyl, 1-Methylpropyl, 2-Methylpropyl, 1,1-Dimethylethyl, Pentyl, Cyclopentyl, 1-Methylbutyl, 2-Methylbutyl, 3-Methylbutyl, 2,2-Dimethylpropyl, 1-Ethylpropyl, Hexyl, Cyclohexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-Methylpropyl sein. Vorzugsweise wird ein Alkohol mit einem C1-C3-Alkylrest, wie z.B. Methyl, Ethyl, Propyl eingesetzt. Besonders bevorzugt ist Ethanol.

Vorzugsweise sind einer, mehrere oder sämtliche der Katalysatoren zur selektiven Isomerisierung von Farnesol zu Nerolidol in Gegenwart von alpha-Bisabolol in der bereit- oder hergestellten Mischung (i) gelöst oder (ii) suspendiert. Der erste Fall (i) stellt eine homogene Katalyse dar, denn Katalysator und Edukte liegen in derselben Phase vor. Der zweite Fall (ii) stellt eine heterogene Katalyse dar, denn Katalysator einerseits und Edukte andererseits liegen in verschiedenen Phasen vor. Im Fall (ii) umfasst der Katalysator bevorzugt ein Trägermaterial. Trägermaterialien sind chemisch inerte Stoffe, die als Unterlage und Gerüst für eine katalytisch aktive Substanz geeignet sind, z. B. großoberflächige Stoffe ausgewählt aus der Gruppe bestehend aus Aktivkohle, Tonerde, Kieselgel, Kieselgur, Talk, Kaolin, Ton, Silicate, oder unter den jeweiligen Reaktionsbedingungen inerte polymere Materialien.

Ein weiterer Gegenstand der vorliegenden Erfindung ist die Verwendung eines Katalysators umfassend eine Verbindung eines Übergangsmetalls der Gruppen 5 bis 8 des Periodensystems, vorzugsweise eine Verbindung eines Übergangsmetalls ausgewählt aus der Gruppe bestehend aus Vanadium, Rhenium, Wolfram, Mangan, Chrom, Molybdän und Palladium zur selektiven Isomerisierung von Farnesol zu Nerolidol in Gegenwart von alpha-Bisabolol. Hinsichtlich bevorzugter Ausführungsformen des erfindungsgemäß einzusetzenden Katalysators gelten dabei die oben im Zusammenhang mit dem erfindungsgemäßen Verfahren gemachten Ausführungen.

Die Erfindung wird nachfolgend anhand von Ausführungsbeispielen näher erläutert.

### Beispiel 1.1: Herstellung einer Mischung umfassend alpha-Bisabolylformiat undFarnesylformiat

In einem 2000 ml-Dreihalskolben mit Rückflusskühler und Thermometer werden 264 g (1,2 mol) (+/-)- Nerolidol und 100 g Hexan vorgelegt. Dann werden bei einer Temperatur im Bereich von 10 bis 15 °C innerhalb von 60 Minuten insgesamt 92 g (2 mol) Ameisensäure zugetropft. Anschließend wird die Reaktionsmischung 20 Stunden lang bei einer Temperatur im Bereich von 10 bis 15 °C gerührt. Die erhaltene Reaktionsmischung wird nach erfolgter gaschromatographischer Analyse (GC) aufgearbeitet. Dazu wird die Reaktionsmischung mit 500 g Wasser versetzt, und danach wird die organische Phase abgetrennt und mit Sodalösung und Wasser neutral gewaschen. Nach dem Abdestillieren des Lösungsmittels verblieben 300 g Rohprodukt.

### Resultate der GC-Analyse

| | |
|---|---|
| alpha-Bisabolylformiate: | 38,9 Gew.-%; |
| Farnesylformiate | 29,7 Gew.-% |

### (Summe der 4 Isomeren cis/cis;cis/trans;trans/cis;trans/trans)

### Anmerkung:

Hier und in den nachfolgenden Beispielen wurden (ohne internen Standard) GC-Flächenprozente bestimmt und in guter Näherung mit den Gewichtsprozenten gleichgesetzt; ein möglicherweise entstandener Fehler wird als vernachlässigbar eingeschätzt.

### Beispiel 1.2: Herstellung einer Mischung umfassend alpha-Bisabolol und Farnesol

In einem 2000 ml-Dreihalskolben mit Rückflusskühler, Tropftrichter und Thermometer werden 300 g (0,8 mol) einer gemäß Beispiel 1.1 hergestellten Mischung umfassend alpha-Bisabolylformiat und Farnesylformiat sowie 400 g Methanol vorgelegt und anschließend bei einer Temperatur von 20 bis 40 °C innerhalb von 15 min mit 480 g Natronlauge (10 gew.-%ig) versetzt. Anschließend wird 2 Stunden lang bei einer Temperatur im Bereich von 30 bis 40 °C nachgerührt. Die erhaltene Reaktionsmischung wird nach erfolgter gaschromatograpischer Analyse (GC) aufgearbeitet. Dazu wird die Reaktionsmischung mit 400 g Wasser und 200 g Diethylether versetzt, und danach wird die organische Phase abgetrennt und mit Wasser neutral gewaschen. Nach dem Abdestillieren des Lösungsmittels verblieben 259,2 g Rohprodukt.

### Resultate der GC-Analyse

| | |
|---|---|
| alpha-Bisabolol: | 37,3% |
| Farnesol: | 28,9% |

### (Summe der 4 Isomere cis/cis;cis/trans;trans/cis;trans/trans)

Dies entspricht in etwa einem Molverhältnis von alpha -Bisabolol zu Farnesol von 1,3 : 1

### GC-Bedingungen:

Gerät: Agilent 6890; FID-Detektor
Säule: 20 m DB-WAX, 0,18 µm Innendurchmesser, 0,18 µm Filmdicke
Trägergas: Wasserstoff, 1,6 bar; Split 1:50
Injektor: 250°C Detektor: 300°C
Temperaturprogramm: 60°C; 8 K/min; 250°C

Durch Rektifkation an einer Kolonne mit 40 Böden konnte der Gehalt an alpha-Bisabolol auf 86 Gew.-% erhöht und der Gehalt an Farnesol auf 7 Gew.-% erniedrigt werden. Dies entspricht einem Molverhältnis von alpha -Bisabolol zu Farnesol von 12,3 : 1.

### Beispiel 2: Herstellung einer Mischung umfassend alpha-Bisabolol und Farnesol und Umsetzen von Farnesol zu Nerolidol in der Mischung (erfindungsgemäß)

In einem 500 ml-Dreihalskolben mit einer 15 bödigen Destillationskolonne, Kolonnenkopf und Thermometer werden 250 g einer Mischung umfassend 43 Gew.-% alpha-Bisabolol, 40 Gew.-% Farnesol (entsprechend 0,45 mol Farnesol, Molverhältnis alpha-Bisabolol zu Farnesol = 1,075) und 1,2 g Vanadyl(IV)-acetylacetonat (entspricht 4,5 mmol) vorgelegt und bei einer Temperatur im Bereich von 174 bis 176 °C und einem Druck von 25 mbar 7 Stunden lang erhitzt. Aus dieser Reaktionsmischung destillieren 119 g einer Fraktion umfassend 76 Gew.-% Nerolidol, 7 Gew.-% alpha-Bisabolol und 7 Gew.-% Kohlenwasserstoffe über.

Die verbleibende Reaktionsmischung wird anschließend durch einfache Destillation bei 1 mbar vom Rückstand getrennt. Hierbei werden 113 g einer Fraktion über Kopf abdestilliert, welche 81 Gew.-% alpha-Bisabolol und 0,7 Gew.-% Farnesol enthält. Dies entspricht einem Molverhältnis von alpha-Bisabolol zu Farnesol von 115 : 1.

### Beispiel 3: Herstellung einer Mischung umfassend alpha-Bisabolol und Farnesol und Umsetzen von Farnesol zu Nerolidol in der Mischung (erfindungsgemäß)

In einem 500 ml-Dreihalskolben mit einer 15 bödigen Destillationskolonne, Kolonnenkopf und Thermometer werden 250 g einer Mischung umfassend 43 Gew.-% alpha-Bisabolol und 40 Gew.-% Farnesol (entsprechend 0,45 mol Farnesol, Molverhältnis alpha-Bisabolol zu Farnesol = 1,075) und 1,1 g Vanadium(V)-oxytriisopropoxid (entspricht 4,5 mmol) vorgelegt und bei einer Temperatur im Bereich von 172 bis 176 °C und einem Druck von 25 mbar 7 Stunden lang erhitzt. Aus dieser Reaktionsmischung destillieren 116 g einer Fraktion umfassend 77 Gew.-% Nerolidol, 6 Gew.-% alpha-Bisabolol und 6 Gew.-% Kohlenwasserstoffe über.

Die verbleibende Reaktionsmischung wird anschließend durch einfache Destillation bei 1 mbar vom Rückstand getrennt. Hierbei werden 119 g einer Fraktion über Kopf abdestilliert, welche 83 Gew.-% alpha-Bisabolol und 1,0 Gew.-% Farnesol enthält. Dies entspricht einem Molverhältnis von alpha-Bisabolol zu Farnesol von 83 : 1.

### Beispiel 4: Herstellung einer Mischung umfassend alpha-Bisabolol und Farnesol und Umsetzen von Farnesol zu Nerolidol in der Mischung (erfindungsgemäß)

In einem 500 ml-Dreihalskolben mit einer 15 bödigen Destillationskolonne, Kolonnenkopf und Thermometer werden 250 g einer Mischung umfassend 43 Gew.-% alpha-Bisabolol und 40 Gew.-% Farnesol (entsprechend 0,45 mol Farnesol, Molverhältnis alpha-Bisabolol zu Farnesol = 1,075) und 1,6 g Vanadium(III)-acetylacetonat (entspricht 4,5 mmol) vorgelegt und bei einer Temperatur von im Bereich von 172 bis 176 °C und einem Druck von 25 mbar 7 Stunden lang erhitzt. Aus dieser Reaktionsmischung destillieren 116 g einer Fraktion umfassend 75 Gew.-% Nerolidol, 6 Gew.-% alpha-Bisabolol und 8 Gew.-% Kohlenwasserstoffe über.

Die verbleibende Reaktionsmischung wird anschließend durch einfache Destillation bei 1 mbar vom Rückstand getrennt. Hierbei werden 107 g einer Fraktion über Kopf abdestilliert, welche 79 Gew.-% alpha-Bisabolol und 0,5 Gew.-% Farnesol enthält. Dies entspricht einem Molverhältnis von alpha-Bisabolol zu Farnesol von 158 : 1.

### Beispiel 5: Herstellung einer Mischung umfassend alpha-Bisabolol und Farnesol und Umsetzen von Farnesol zu Nerolidol in der Mischung (erfindungsgemäß)

In einem 500 ml Dreihalskolben mit einer 15 bödigen Destillationskolonne, Kolonnenkopf und Thermometer werden 250 g einer Mischung umfassend 43 Gew.-% alpha-Bisabolol und 40 Gew.-% Farnesol (entsprechend 0,45 mol Farnesol, Molverhältnis alpha-Bisabolol zu Farnesol = 1,075) und 0,5 g Ammoniummetavanadat (entspricht 4,5 mmol) vorgelegt und bei einer Temperatur im Bereich von 173 bis 176 °C und einem Druck von 25 mbar 7 Stunden lang erhitzt. Aus dieser Reaktionsmischung destillieren 132 g einer Fraktion umfassend 65 Gew.-% Nerolidol, 15 Gew.-% alpha-Bisabolol und 6 Gew.-% Kohlenwasserstoffe über.

Die verbleibende Reaktionsmischung wird anschließend durch einfache Destillation bei 1 mbar vom Rückstand getrennt. Hierbei werden 102 g einer Fraktion über Kopf abdestilliert, welche 85 Gew.-% alpha-Bisabolol und 0,9 Gew.-% Farnesol enthält. Dies entspricht einem Molverhältnis von alpha-Bisabolol zu Farnesol von 94,5 : 1.

### Beispiel 6: Herstellung einer Mischung umfassend alpha-Bisabolol und Farnesol und Umsetzen von Farnesol zu Nerolidol in der Mischung (erfindungsgemäß)

In einem 50 ml Erlenmeyerkolben werden 12,5 g Wolframsäure (Wolfram(VI)oxid-Monohydrat; entspricht 50 mmol) in 20 g Wasserstoffperoxid (30 Gew.-%ig) suspendiert und bei einer Temperatur von 40 °C für 6 Stunden gerührt. Nach Filtration wird in ein Reagenzglas überführt und fein verteilter Feststoff über Nacht absitzen gelassen. 20 g der über dem abgesetzten Feststoff überstehenden Lösung (entspricht ∼20 mmol) werden zu 750 g einer Mischung umfassend 10 Gew.-% Kohlenwasserstoffe, 40 Gew.-% alpha-Bisabolol und 35 Gew.-% Farnesol (entsprechend 1,18 mol Farnesol, Molverhältnis alpha-Bisabolol zu Farnesol = 1,14) in einen 2000 ml Dreihalskolben mit einer 15 bödigen Destillationskolonne, Kolonnenkopf und Thermometer überführt und anschließend mit einer Lösung von 10 g 8-Hydroxychinolin (69 mmol) in 20 g Ethanol versetzt. Danach wird bei verringertem Druck eine Fraktion umfassend Alkohol und 8-Hydroxychinolin abdestilliert.

Anschließend wird die Reaktionsmischung bei einer Sumpftemperatur im Bereich von 155 bis 160 °C und einem Druck von 14 mbar für 5 Stunden erhitzt. Es destillierten 438 g einer Fraktion umfassend 56 Gew.-% Nerolidol, 10 Gew.-% alpha-Bisabolol und 24 Gew.-% Kohlenwasserstoffe über. Im Sumpf der Reaktionsmischung verblieben 317 g eines Materials, welches einen mittels Gaschromatographie bestimmten Gehalt von 77 Gew.-% alpha-Bisabolol und 1,5 Gew.-% Farnesol aufweist. Dies entspricht einem Molverhältnis von alpha-Bisabolol zu Farnesol von 51,3 : 1.

Der Sumpf der Reaktionsmischung wird mit 300 g Cyclohexan versetzt und über Talkum filtriert. Nach Entfernung des Cyclohexans werden durch einfache Destillation des Sumpfes an einer Destillationsbrücke 263 g eine Fraktion umfassend 81 Gew.-% alpha-Bisabolol, <0,1 Gew.-% Farnesol, 1,5 Gew.-% Nerolidol und 7 Gew.-% Kohlenwasserstoffe erhalten. Dies entspricht einem Molverhältnis von alpha-Bisabolol zu Farnesol von > 810: 1.

### Beispiel 7: Herstellung einer Mischung umfassend alpha-Bisabolol und Farnesol und Umsetzen von Farnesol zu Nerolidol in der Mischung in einer Eintopfreaktion (erfindungsgemäß)

In einen 2 L Dreihalskolben mit einer 15bödigen Destillationskolonne, Kolonnenkopf und Thermometer werden 1000 g einer gemäß Beispiel 1.1 hergestellten Mischung umfassend alpha-Bisabolylformiat und Farnesylformiat vorgelegt und mit 276 g Ethanol und 10 g Tetraethyltitanat versetzt. Anschließend werden bei Normaldruck zunächst Ethylformiat und dann Ethanol abdestilliert.

In dem Dreihalskolben werden 887 g einer Mischung umfassend 13 Gew.-% Kohlenwasserstoffe, 40 Gew.-% alpha-Bisabolol und 30 Gew.-% Farnesol (entsprechend 1,2 mol Farnesol, Molverhältnis alpha-Bisabolol zu Farnesol = 1,33) erhalten, welche anschließend mit 4,0 g Vanadyl(IV)-acetylacetonat versetzt und bei einer Temperatur im Bereich von 174 bis 176 °C und einem Druck von 25 mbar 7 Stunden lang erhitzt wird. Aus dieser Reaktionsmischung destillieren 296 g einer Fraktion umfassend 71 Gew.-% Nerolidol und 12 Gew.-% alpha-Bisabolol über.

Die Umesterung von alpha-Bisabolylformiat und Farnesylformiat mit Ethanol zu Ethylformiat, alpha-Bisabolol und Farnesol sowie die anschließende katalytische Umsetzung von Farnesol in Gegenwert von alpha-Bisabol zu Nerolidol erfolgen somit in situ als Eintopfreaktion.

Die verbleibende Reaktionsmischung wird anschließend durch einfache Destillation bei 1 mbar vom Rückstand getrennt. Hierbei werden 399 g einer Fraktion erhalten, welche 74 Gew.-% alpha-Bisabolol und 1,4 Gew.-% Farnesol enthält. Dies entspricht einem Molverhältnis von alpha-Bisabolol zu Farnesol von 52,8 : 1.

## Patentansprüche

1. Verfahren zum Umsetzen von Farnesol zu Nerolidol in Gegenwart von alpha-Bisabolol, mit folgenden Schritten:
- Bereitstellen oder Herstellen einer Mischung umfassend
- alpha-Bisabolol,
- Farnesol,
- einen oder mehrere Katalysatoren zur selektiven Isomerisierung von Farnesol zu Nerolidol in Gegenwart von alpha-Bisabolol
- sowie gegebenenfalls weitere Bestandteile,
- Umsetzen von Farnesol zu Nerolidol in der Mischung.

2. Verfahren nach Anspruch 1, wobei die Verfahrensbedingungen so gewählt sind, dass nach dem Umsetzen von Farnesol zu Nerolidol das Molverhältnis von alpha-Bisabolol zu Farnesol größer ist als in der bereitgestellten oder hergestellten Mischung,

3. Verfahren nach Anspruch 1 oder 2, wobei in der bereitgestellten oder hergestellten Mischung das Molverhältnis von alpha-Bisabolol zu Farnesol in einem Bereich von 15:1 bis 1:100, vorzugsweise 2 : 1 bis 1 : 2 liegt.

4. Verfahren nach Anspruch 1, 2 oder 3, wobei die Verfahrensbedingungen so gewählt sind, dass nach dem Umsetzen von Farnesol zu Nerolidol das Molverhältnis von alpha-Bisabolol zu Farnesol 30 : 1 oder höher ist, bevorzugt 50 : 1 oder höher, weiter bevorzugt 80 : 1 oder höher, besonders bevorzugt 100 : 1 oder höher.

5. Verfahren nach einem der vorhergehenden Ansprüche, wobei einer, mehrere oder sämtliche der Katalysatoren zur selektiven Isomerisierung von Farnesol zu Nerolidol in Gegenwart von alpha-Bisabolol eine Verbindung eines Übergangsmetalls der Gruppen 5 bis 8 des Periodensystems umfassen, vorzugsweise eine Verbindung eines Übergangsmetalls ausgewählt aus der Gruppe bestehend aus Vanadium, Rhenium, Wolfram, Mangan, Chrom, Molybdän und Palladium.

6. Verfahren nach einem der vorhergehenden Ansprüche, wobei einer, mehrere oder sämtliche der Katalysatoren zur selektiven Isomerisierung von Farnesol zu Nerolidol in Gegenwart von alpha-Bisabolol ausgewählt sind aus der Gruppe bestehend aus:
- Vanadiumsäure und deren Ester und Ammoniumsalze, vorzugsweise ausgewählt aus der Gruppe bestehend aus Vanadium(III)-acetylacetonat, Vanadyl(IV)-acetylacetonat, Vanadium(V)-oxytriisopropoxid und Ammoniummetavanadat
- Chelate und Organometallverbindungen der Übergangsmetalle der Gruppen 5 bis 8 des Periodensystems, vorzugsweise Chelate und Organometallverbindungen der Übergangsmetalle ausgewählt aus der Gruppe bestehend aus Vanadium, Rhenium, Wolfram, Mangan, Chrom, Molybdän und Palladium, vorzugsweise ausgewählt aus der Gruppe der Oxoperoxowolfram(VI)-Komplexe umfassend einen oder mehrere Liganden in Form von 8-Hydroxychinolin.

7. Verfahren nach einem der vorangehenden Ansprüche, wobei die bereitgestellte oder hergestellte Mischung bereitgestellte oder hergestellte Mischung 0,0001 bis 1 Mol, vorzugsweise 0,001 bis 0,5 Mol, an Übergangsmetall pro Mol Farnesol enthält, bezogen auf die in der bereitgestellten oder hergestellten Mischung vorhandene Stoffmenge an Farnesol.

8. Verfahren nach einem der vorangehenden Ansprüche, wobei das Umsetzen von Farnesol zu Nerolidol bei einer Temperatur im Bereich von 50 bis 300 °C durchgeführt wird, vorzugsweise im Bereich von 150 bis 200 °C und/oder wobei das Umsetzen von Farnesol zu Nerolidol bei einem Druck von weniger als 1000 mbar, vorzugsweise im Bereich vom 0,1 bis 500 mbar durchgeführt wird.

9. Verfahren nach einem der vorangehenden Ansprüche, wobei in der bereitgestellten oder hergestellten Mischung ein oder mehrere weitere Bestandteile vorhanden sind, die ausgewählt sind aus der Gruppe bestehend aus: Nerolidol, Eliminierungsprodukte des alpha-Bisabolols, Eliminierungsprodukte des Farnesols, Eliminierungsprodukte des Nerolidols, Veretherungsprodukte des Farnesols, weitere Sesquiterpene, Sesquiterpenalkohole, Germacradienol, beta-Bisabolol, weitere Umlagerungsprodukte von alpha-Bisabolol, Nerolidol und Farnesol, sonstige Lösungsmittel.

10. Verfahren nach einem der vorangehenden Ansprüche, wobei der Schritt des Herstellens der Mischung das Umsetzen von Nerolidol zu einer alpha-Bisabolol und Farnesol umfassenden Mischung umfasst.

11. Verfahren nach einem der vorangehenden Ansprüche, wobei während und/oder nach dem Umsetzen des Farnesols zu Nerolidol alpha-Bisabolol und Nerolidol voneinander getrennt werden, vorzugsweise indem während und/oder nach dem Umsetzen des Farnesols zu Nerolidol ein destillatives Abtrennen des Nerolidols aus der durch Umsetzen des Farnesols zu Nerolidol erhaltenen Mischung erfolgt.

12. Verfahren nach einem der vorangehenden Ansprüche, wobei die Schritte
- Herstellen einer Mischung umfassend
- alpha-Bisabolol,
- Farnesol,
- einen oder mehrere Katalysatoren zur selektiven Isomerisierung von Farnesol zu Nerolidol in Gegenwart von alpha-Bisabolol
- sowie gegebenenfalls weitere Bestandteile,
und
- Umsetzen von Farnesol zu Nerolidol in der Mischung in demselben Reaktionsgefäß ausgeführt werden.

13. Verfahren nach einem der vorangehenden Ansprüche, wobei der Schritt des Bereitstellens oder Herstellens der Mischung das
- Verseifen von alpha-Bisabolylformiat und Farnesylformiat in Mischung miteinander
oder
- Umestern von alpha-Bisabolylformiat und Farnesylformiat in Mischung miteinander mit einem Alkohol
umfasst.

14. Verfahren nach einem der vorangehenden Ansprüche, wobei der Katalysator in der bereit- oder hergestellten Mischung
- gelöst ist
oder
- suspendiert ist, wobei in diesem Falle der Katalysator bevorzugt ein Trägermaterial umfasst.

15. Verwendung eines Katalysators umfassend eine Verbindung eines Übergangsmetalls der Gruppen 5 bis 8 des Periodensystems, vorzugsweise eine Verbindung eines Übergangsmetalls ausgewählt aus der Gruppe bestehend aus Vanadium, Rhenium, Wolfram, Mangan, Chrom, Molybdän und Palladium zur selektiven Isomerisierung von Farnesol zu Nerolidol in Gegenwart von alpha-Bisabolol.

## Claims

1. Method of converting farnesol to nerolidol in the presence of alpha-bisabolol, with the following steps:
- providing or preparing a mixture comprising
- alpha-bisabolol,
- farnesol,
- one or more catalysts for selective isomerization of farnesol to nerolidol in the presence of alpha-bisabolol
- and optionally further constituents,
- converting farnesol to nerolidol in the mixture.

2. Method according to claim 1, wherein the process conditions are selected so that after conversion of farnesol to nerolidol the molar ratio of alpha-bisabolol to farnesol is higher than in the mixture that was provided or prepared.

3. Method according to claim 1 or 2, wherein in the mixture that was provided or prepared, the molar ratio of alpha-bisabolol to farnesol is in a range from 15 : 1 to 1 : 100, preferably 2 : 1 to 1 : 2.

4. Method according to claim 1, 2 or 3, wherein the process conditions are selected so that after conversion of farnesol to nerolidol the molar ratio of alpha-bisabolol to farnesol is 30 : 1 or higher, preferably 50 : 1 or higher, further preferably 80 : 1 or higher, especially preferably 100 : 1 or higher.

5. Method according to one of the preceding claims, wherein one, several or all of the catalysts for selective isomerization of farnesol to nerolidol in the presence of alpha-bisabolol comprise a compound of a transition metal of groups 5 to 8 of the periodic table, preferably a compound of a transition metal selected from the group consisting of vanadium, rhenium, tungsten, manganese, chromium, molybdenum and palladium.

6. Method according to one of the preceding claims, wherein one, several or all of the catalysts for selective isomerization of farnesol to nerolidol in the presence of alpha-bisabolol are selected from the group consisting of:
- vanadic acid and esters and ammonium salts thereof, preferably selected from the group consisting of vanadium(III) acetylacetonate, vanadyl(IV) acetylacetonate, vanadium(V) oxytriisopropoxide and ammonium metavanadate
- chelates and organometallic compounds of the transition metals of groups 5 to 8 of the periodic table, preferably chelates and organometallic compounds of the transition metals selected from the group consisting of vanadium, rhenium, tungsten, manganese, chromium, molybdenum and palladium, preferably selected from the group of oxoperoxotungsten(VI) complexes comprising one or more ligands in the form of 8-hydroxyquinoline.

7. Method according to one of the preceding claims, wherein the mixture that was provided or prepared contains 0.0001 to 1 mol, preferably 0.001 to 0.5 mol, of transition metal per mol of farnesol relative to the amount of farnesol present in the mixture that was provided or prepared.

8. Method according to one of the preceding claims, wherein the conversion of farnesol to nerolidol is carried out at a temperature in the range from 50 to 300°C, preferably in the range from 150 to 200°C and/or wherein the conversion of farnesol to nerolidol is carried out at a pressure of less than 1000 mbar, preferably in the range from 0.1 to 500 mbar.

9. Method according to one of the preceding claims, wherein in the mixture that was provided or prepared one or more further constituents are present, which are selected from the group consisting of: nerolidol, elimination products of alpha-bisabolol, elimination products of farnesol, elimination products of nerolidol, etherification products of farnesol, further sesquiterpenes, sesquiterpene alcohols, germacradienol, beta-bisabolol, further rearrangement products of alpha-bisabolol, nerolidol and farnesol, other solvents.

10. Method according to one of the preceding claims, wherein the step of preparing the mixture comprises the conversion of nerolidol to a mixture comprising alpha-bisabolol and farnesol.

11. Method according to one of the preceding claims, wherein during and/or after conversion of farnesol to nerolidol, alpha-bisabolol and nerolidol are separated from one another, preferably by separation by distillation of nerolidol from the mixture obtained by conversion of farnesol to nerolidol during and/or after conversion of farnesol to nerolidol.

12. Method according to one of the preceding claims, wherein the steps
- preparing a mixture comprising
- alpha-bisabolol,
- farnesol,
- one or more catalysts for selective isomerization of farnesol to nerolidol in the presence of alpha-bisabolol
- and optionally further constituents,
and
- converting farnesol to nerolidol in the mixture
are carried out in the same reaction vessel.

13. Method according to one of the preceding claims, wherein the step of providing or preparing the mixture comprises
- saponification of alpha-bisabolyl formate and farnesyl formate mixed together
or
- transesterification of alpha-bisabolyl formate and farnesyl formate mixed together with an alcohol.

14. Method according to one of the preceding claims, wherein the catalyst in the mixture that is provided or prepared
- is dissolved
or
- is suspended, wherein in this case the catalyst preferably comprises a support material.

15. Use of a catalyst comprising a compound of a transition metal of groups 5 to 8 of the periodic table, preferably a compound of a transition metal selected from the group consisting of vanadium, rhenium, tungsten, manganese, chromium, molybdenum and palladium for selective isomerization of farnesol to nerolidol in the presence of alpha-bisabolol.

## Revendications

1. Procédé de conversion de farnésol en nérolidol en présence d'alpha-bisabolol, comprenant les étapes suivantes:
- fournir ou préparer un mélange comprenant
- l'alpha-bisabolol,
- le farnésol,
- un ou plusieurs catalyseurs pour l'isomérisation sélective de farnésol en nérolidol en présence d'alpha-bisabolol,
- ainsi que, le cas échéant, d'autres composants,
- convertir le farnésol en nérolidol dans le mélange.

2. Procédé selon la revendication 1, dans lequel les conditions de procédé sont choisies de manière à ce que, après la conversion de farnésol en nérolidol, le rapport molaire de l'alpha-bisabolol au farnésol soit supérieur à celui dans le mélange fourni ou préparé.

3. Procédé selon la revendication 1 ou 2, dans lequel, dans ledit mélange fourni ou préparé, le rapport molaire de l'alpha-bisabolol au farnésol est compris entre 15 : 1 et 1 : 100, de préférence entre 2 : 1 à 1 : 2.

4. Procédé selon la revendication 1, 2 ou 3, dans lequel les conditions de procédé sont choisies de manière à ce que, après la conversion de farnésol en nérolidol, le rapport molaire de l'alpha-bisabolol au farnésol soit de 30 : 1 ou plus, de préférence de 50 : 1 ou plus, de préférence encore de 80 : 1 ou plus, de manière particulièrement préférée de 100 : 1 ou plus.

5. Procédé selon l'une quelconque des revendications précédentes, dans lequel un, plusieurs ou l'ensemble des catalyseurs pour l'isomérisation sélective de farnésol en nérolidol en présence d'alpha-bisabolol comprennent un composé d'un métal transitoire des groupes 5 à 8 de la classification périodique des éléments, de préférence un composé d'un métal transitoire choisi dans le groupe constitué par le vanadium, le rhénium, le tungstène, le manganèse, le chrome, le molybdène et le palladium.

6. Procédé selon l'une quelconque des revendications précédentes, dans lequel un, plusieurs ou l'ensemble des catalyseurs pour l'isomérisation sélective de farnésol en nérolidol en présence d'alpha-bisabolol sont choisis dans le groupe constitué par :
- l'acide vanadique et ses esters et sels d'ammonium, de préférence choisis dans le groupe constitué par l'acétylacétonate de vanadium(III), l'acétylacétonate de vanadyle(IV), l'oxytriisopropoxyde de vanadium(V) et le métavanadate d'ammonium,
- les chélates et les composés organométalliques des métaux transitoires des groupes 5 à 8 de la classification périodique des éléments, de préférence les chélates et les composés organométalliques des métaux transitoires choisis dans le groupe constitué par le vanadium, le rhénium, le tungstène, le manganèse, le chrome, le molybdène et le palladium, de préférence choisis dans le groupe des complexes d'oxoperoxo tungstène(VI) comprenant un ou plusieurs ligands sous forme de 8-hydroxyquinoléine.

7. Procédé selon l'une quelconque des revendications précédentes, dans lequel le mélange fourni ou préparé contient de 0,0001 à 1 mol, préférence de 0,001 à 0,5 mol de métal transitoire par mol de farnésol, par rapport à la quantité de substance de farnésol présente dans le mélange fourni ou préparé.

8. Procédé selon l'une quelconque des revendications précédentes, dans lequel la conversion de farnésol en nérolidol est effectuée à une température comprise entre 50 et 300 °C, de préférence comprise entre 150 et 200 °C, et/ou dans lequel la conversion de farnésol en nérolidol est effectuée à une pression inférieure à 1000 mbar, de préférence comprise entre 0,1 et 500 mbar.

9. Procédé selon l'une quelconque des revendications précédentes, dans lequel, dans ledit mélange fourni ou préparé est/sont présent(s) un ou plusieurs autre(s) composant(s) qui sont choisis dans le groupe constitué par : le nérolidol, les produits d'élimination de l'alpha-bisabolol, les produits d'élimination du farnésol, les produits d'élimination du nérolidol, les produits d'éthérification du farnésol, d'autres sesquiterpènes, les alcools sésquiterpéniques, le germacradiénol, le béta-bisabolol, d'autres produits de transposition d'alpha-bisabolol, de nérolidol et de farnésol, d'autres solvants.

10. Procédé selon l'une quelconque des revendications précédentes, dans lequel l'étape de préparation du mélange comprend la conversion de nérolidol en un mélange comprenant l'alpha-bisabolol et le farnésol.

11. Procédé selon l'une quelconque des revendications précédentes, dans lequel, durant et/ou après la conversion du farnésol en nérolidol, l'alpha-bisabolol et le nérolidol sont séparés l'un de l'autre, de préférence en réalisant, durant et/ou après la conversion du farnésol en nérolidol, une séparation par voie de distillation du nérolidol dudit mélange obtenu par conversion du farnésol en nérolidol.

12. Procédé selon l'une quelconque des revendications précédentes, dans lequel les étapes consistant
- à préparer un mélange comprenant
- l'alpha-bisabolol,
- le farnésol,
- un ou plusieurs catalyseurs pour l'isomérisation sélective de farnésol en nérolidol en présence d'alpha-bisabolol
- ainsi que, le cas échéant, d'autres composants,
et
- à convertir le farnésol en nérolidol dans le mélange sont mises en oeuvre dans le même réacteur.

13. Procédé selon l'une quelconque des revendications précédentes, dans lequel l'étape consistant à fournir ou à préparer le mélange comprend
- la saponification de formiate d'alpha-bisabolyle et de formiate de farnésyle en mélange l'un avec l'autre
ou
- la transestérification de formiate d'alpha-bisabolyle et de formiate de farnésyle en mélange l'un avec l'autre, avec un alcool.

14. Procédé selon l'une quelconque des revendications précédentes, dans lequel le catalyseur est
- dissous
ou
- mis en suspension dans le mélange fourni ou préparé, dans ce cas, le catalyseur comprenant, de préférence, une matière support.

15. Utilisation d'un catalyseur comprenant un composé d'un métal transitoire des groupes 5 à 8 de la classification périodique des éléments, de préférence un composé d'un métal transitoire choisi dans le groupe constitué par le vanadium, le rhénium, le tungstène, le manganèse, le chrome, le molybdène et le palladium, pour l'isomérisation sélective de farnésol en nérolidol en présence d'alpha-bisabolol.
